# EUROPEAN PATENT APPLICATION

(11) **EP 4 035 727 A1**
(43) Date of publication of application: **03.08.2022**
(21) Application number: 20867922.5
(22) Date of filing: 27.09.2020
(51) Int. Cl.: A61N 1/362, A61N 1/372, A61N 1/375

(54) **LEADLESS PACEMAKER, HEAD END COMPONENT, TAIL END COMPONENT, AND DELIVERY DEVICE**

(30) Priority: 26.09.2019 CN 201910918535
(71) Applicant: MicroPort Soaring CRM (Shanghai) Co., Ltd., Shanghai 201203 (CN)
(72) Inventor: CHENG, Zhijun, Shanghai 201203 (CN); JANG, Grace, Shanghai 201203 (CN)
(74) Representative: Lavoix
(86) International application number: PCT/CN2020/118091
(87) International publication number: WO 2021/057968

(57) **Abstract**

A leadless pacemaker, a leading component, a trailing component and a delivery device are disclosed. The leading component (10) includes a leading end body (100), a first connection member (101) and a second connection member (102). The trailing component (20) includes a trailing end body (200), a third connection member (203) and a fourth connection member (204). When the leading component (10) is connected to or removed from the trailing component (20), the first connection member (101) is connected to the delivery device (30); the leading component (10) can be axially immobilized by the delivery device, and force is applied to the fourth connection member (204) by the delivery device (30) to enable the second connection member (102) to be connected to or separated from the third connection member (203), so that the leading component (10) and the trailing component (20) of the leadless pacemaker can be connected or separated conveniently; in addition, during performing separation and connection, the leading component (10) can be fixed by the delivery device (30) to prevent the leading component (10) from pulling the heart tissue, thereby making separation and connection safer. When the battery of the leadless pacemaker is exhausted, the connection relationship between the leading component (10) and the heart can be kept unchanged, and the trailing component (20) can be conveniently replaced.

## Description

### TECHNICAL FIELD

The present invention relates to the field of medical devices and, in particular, to a leadless pacemaker, a leading component, a trailing component and a delivery device thereof.

### BACKGROUND

Cardiac pacemakers are implantable therapeutic electronic devices incorporating an impulse generator for delivering electrical impulses which are transferred via leads to electrodes to stimulate the heart muscle, to which the electrodes are attached, thus causing the heart to contract and effecting treatment of heart dysfunction caused by some cardiac arrhythmia conditions. However, traditional lead cardiac pacemakers require the creation of pockets where they are to be inserted and long-term presence of their leads in veins and therefore suffer from a high incidence of pocket- or lead-related complications, which present a serious threat to the life and health of patients. By contrast, leadless pacemakers integrate a battery, circuitry and pacing electrodes in a "compact capsule" that can be overall implanted into the heart simply via a percutaneous catheter, and have found extensive use in clinical practice thanks to a wide range of advantages including ease of operation, high convenience, minimal trauma, eliminated need for surgical creation of a pacemaker pocket, unaffected patients' appearance and absence of pacemaker pocket- or lead-related complications.

Conventional leadless pacemakers typically includes a fixation feature disposed at the leading end, which can pierce the myocardium when the pacemaker has been delivered through a delivery sheath to a target site within the heart, thus attaching the pacemaker to the myocardium. However, after the leadless pacemaker is implanted, the fixation feature will be wrapped or encapsulated by myocardial tissue, disallowing removal of the leadless pacemaker. Therefore, when its battery runs out, a new pacemaker has to be implanted, with the old one having to be still retained in the heart. Consequently, in addition to continued occupation of the intracardiac space, the old leadless pacemaker with the depleted battery may adversely affect the new pacemaker. Further, the implantation of the new pacemaker may require the creation of another incision in the hart, bringing additional damage to the patient.

### SUMMARY OF THE INVENTION

It is an objective of the present invention to overcome the problem of difficult replacement after battery depletion of conventional leadless pacemakers by presenting a novel leadless pacemaker, a leading component, a trailing component and a delivery device thereof.

To this end, in a first aspect of the present invention, there is provided a leading component of a leadless pacemaker, configured for detachably connectable to a trailing component of the leadless pacemaker, the leading component including:
a leading end body configured for anchoring to a first object;
a first connection member disposed around the leading end body, the first connection member configured for detachably connectable to a delivery device; and
a second connection member disposed at a proximal end of the leading end body, the second connection member configured for detachably connectable to the trailing component.

Optionally, in the leading component, the attachment of the first connection member to the delivery device may restrict at least axial movement of the first connection member relative to the delivery device.

Optionally, in the leading component, the first connection member may include a first threaded section or a first circumferential snap fastener.

Optionally, in the leading component, the second connection member may include a first projection projecting from a proximal end face of the leading end body or a first cavity provided at the proximal end of the leading end body, the first projection or cavity configured to mate with a corresponding connection structure of the trailing component.

Optionally, in the leading component, the second connection member may further include a first locking element provided on a side wall of the first projection or on a side wall of the first cavity.

Optionally, the leading component may further include a fixation member, which is fixed to the leading end body at one end and configured to pierce and anchor in the first object at the other end.

In a second aspect of the present invention, there is provided a trailing component of a leadless pacemaker, configured for detachably connectable to a leading component of the leadless pacemaker, the trailing component including:
a trailing end body;
a third connection member disposed at a distal end of the trailing end body, the third connection member configured for detachably connectable to the leading component; and
a fourth connection member disposed at a proximal end of the trailing end body, the fourth connection member configured for detachably connectable to a delivery device,
the fourth connection member configured to be able to move in an axial direction of the trailing end body relative to the third connection member when driven by the delivery device.

Optionally, in the trailing component, the third connection member may include a second projection projecting from a distal end face of the trailing end body or a second cavity provided at the distal end of the trailing end body, the second projection or cavity configured to mate with a corresponding connection structure of the leading component.

Optionally, in the trailing component, the third connection member may further include a second locking element provided on a side wall of the second projection or on a side wall of the second cavity, the second locking element configured to lock the leading component.

Optionally, in the trailing component, the locking element may be annular.

Optionally, the trailing component may further include a limiting block, wherein in the case of the third connection member including the second cavity, the limiting block is movably disposed in the trailing end body. Alternatively, in the case of the third connection member including the second projection, the limiting block is movably disposed in the second projection. The limiting block may be configured to limit the second locking element.

Optionally, in the trailing component, the limiting block may be coupled to the fourth connection member so that the fourth connection member is able to drive the limiting block to move in the axial direction of the trailing end body to or away from a position where it limits the locking element.

Optionally, in the trailing component, the fourth connection member may be coupled to the trailing end body, movable in the axial direction of the trailing end body and configured to drive the limiting block to move under the action of an axial force exerted by the delivery device.

Optionally, in the trailing component, when the axial force is exerted on the fourth connection member by the delivery device away from the trailing end body, the limiting block may move away from the position where it limits the locking element.

Optionally, in the trailing component, the limiting block may be tied to the fourth connection member by a flexible thread.

Optionally, the trailing component may further include a first force-applying element configured to exert a distal first force on the limiting block, which tends to maintain the limiting block at the position where it limits the locking element, wherein the fourth connection member has to overcome the first force before it can move the limiting block away from the position where it limits the locking element.

Optionally, in the trailing component, the first force-applying element may be an elastic element.

Optionally, in the trailing component, a slide channel in which the limiting block is slidably disposed may be provided in the trailing end body or in the second projection.

Optionally, in the trailing component, the slide channel may be provided with a first limiting surface, wherein in the case of the third connection member including the second cavity, the slide channel is provided axially in the trailing end body and the first limiting surface is configured to abut against the limiting block and thus prevent the limiting block from axially moving beyond the distal end of the trailing end body.

Alternatively, in the case of the third connection member including the second projection, the slide channel may be provided axially in the second projection and the first limiting surface may be configured to abut against the limiting block and thus prevent the limiting block from axially moving beyond a distal end of the second projection.

Optionally, in the trailing component, the fourth connection member may include a second threaded section or a second circumferential snap fastener.

In a third aspect of the present invention, there is provided a leadless pacemaker including a leading component and a trailing component,
the leading component including a leading end body, a first connection member and a second connection member, the leading end body configured for anchoring to a first object, the first connection member disposed around the leading end body and configured for detachably connectable to a delivery device, the second connection member disposed at a proximal end of the leading end body,
the trailing component including a trailing end body, a third connection member and a fourth connection member, the third connection member disposed at a distal end of the trailing end body, the fourth connection member disposed at a proximal end of the trailing end body and configured for detachably connectable to the delivery device, the fourth connection member configured to be able to move in an axial direction of the trailing end body relative to the third connection member when driven by the delivery device,
wherein the leading component is detachably attachable to the trailing component with the aid of the second and third connection members.

Optionally, in the leadless pacemaker, the second connection member of the leading component may include a first projection and the third connection member of the trailing component may include a second cavity adapted to receive the first projection. Alternatively, the second connection member may include a first cavity and the third connection member of the trailing component may include a second projection adapted to be received in the first cavity.

Optionally, in the leadless pacemaker, the second connection member may further include a first locking element provided on a side wall of the first projection or on a side wall of the first cavity, wherein the third connection member further includes a second locking element provided on a side wall of the second projection or on a side wall of the second cavity, and wherein one of the first and second locking elements has a snap groove, and the other of them has a snap rib adapted to snap in the groove.

Optionally, in the leadless pacemaker, the first and second locking elements may be electrical conductors. Alternatively, the first projection and the second cavity may be electrical conductors. Alternatively, the second projection and the first cavity may be electrical conductors.

In a fourth aspect of the present invention, there is provided a delivery device for delivering the leading component as defined above, the trailing component as defined above, or the leadless pacemaker as defined above, the delivery device including:
a first sheath configured to engage with the first connection member of the leading component, the first sheath configured to axially immobilize the leading component; and
a delivery rod disposed inside the first sheath, the delivery rod configured to engage with the fourth connection member of the trailing component, the delivery rod configured to attach or detach the trailing component to or from the leading component.

Optionally, the delivery device may further include a second sheath disposed between the first sheath and the delivery rod, the second sheath configured to abut against the trailing end body and thus restrict its axial proximal movement.

In summary, the present invention provides a leadless pacemaker, a leading component, a trailing component and a delivery device. The leading component includes a leading end body, a first connection member and a second connection member. The trailing component includes a trailing end body, a third connection member and a fourth connection member. Attachment or detachment of the leading and trailing components of the leadless pacemaker can be easily accomplished by coupling the first connection member to the delivery device and then, with the leading component being axially immobilized by the delivery device, coupling or decoupling the second connection member to or from the third connection member under the action of an axial force exerted on the fourth connection member by the delivery device. During the attachment or detachment, through axially immobilizing the leading component using the delivery device, pulling or retracting of cardiac tissue to which the leading component is anchored is avoided, making the attachment or detachment safer. In this way, when a battery in the trailing component is exhausted, the leading component is allowed to remain in the heart, while the trailing component can be conveniently replaced with a new one using the delivery device without affecting the leading component at all.

### BRIEF DESCRIPTION OF THE DRAWINGS

Those of ordinary skill in the art would appreciate that the following drawings are presented merely to enable a better understanding of the present invention rather than to limit the scope thereof in any sense. In the drawings:
Fig. 1 is a schematic illustration of a leading component according to an embodiment of the present invention;
Fig. 2 is a schematic illustration of a trailing component according to an embodiment of the present invention;
Fig. 3 is a schematic illustration of a leadless pacemaker according to an embodiment of the present invention;
Fig. 4 is a schematic illustration of a delivery device according to an embodiment of the present invention;
Fig. 5 is another schematic illustration of the delivery device of Fig. 4 with a delivery rod thereof extending out;
Fig. 6 schematically illustrates a first step in a trailing component replacement process performed by the delivery device according to an embodiment of the present invention;
Fig. 7 schematically illustrates a second step in the trailing component replacement process of Fig. 6;
Fig. 8 schematically illustrates a third step in the trailing component replacement process of Fig. 6;
Fig. 9 schematically illustrates a fourth step in the trailing component replacement process of Fig. 6; and
Fig. 10 schematically illustrates the replacement of a trailing component in the leadless pacemaker according to another embodiment of the present invention.

In these figures,
10-leading component; 100-leading end body; 101-first connection member; 102-second connection member; 1021-first locking element; 1022-first projection; 1023-first cavity; 103-fixation member; 104-electrode;
20-trailing component; 200-trailing end body; 201-limiting block; 202-first force-applying element; 203-third connection member; 2031-second locking element; 2032-second cavity; 2033-second projection; 204-fourth connection member; 2041-second threaded section; 2042-fourth connection member body; 2043-guide slope; 205-flexible thread; 206-sealing ring; 207-sensing electrode; 208-second limiting surface; 209-first limiting surface; 210-slide channel;
30-delivery device; 301-first sheath; 302-second sheath; 303-delivery rod; 304-third threaded section; 305-fourth threaded section;
501-cardiac tissue; 502-proliferating tissue.

### DETAILED DESCRIPTION

Objects, features and advantages of the present invention will become more apparent upon reading the following more detailed description of the present invention, which is set forth by way of particular embodiments with reference to the accompanying drawings. Note that the figures are provided in a very simplified form not necessarily drawn to exact scale for the only purpose of helping to explain the disclosed embodiments in a more convenient and clearer way. In addition, structures shown in the figures are usually part of actual structures. In particular, as the figures tend to have distinct emphases, they are often drawn to different scales.

As used herein and in the appended claims, the singular forms "a", "an" and "the" include plural referents, unless the context clearly dictates otherwise. As used herein and in the appended claims, the term "or" is generally employed in the sense of "and/or", unless the context clearly dictates otherwise. In general, the term "proximal" refers to an end closer to an operator, while the term "distal" refers to an end closer to an implant.

The core idea of the present invention is to provide a leadless pacemaker, a leading component, a trailing component and a delivery device. The leading component includes a leading end body, a first connection member and a second connection member and is configured for anchoring to a first object. The first connection member is disposed around the leading end body and is configured for detachable attachment to the delivery device. The second connection member is disposed at a proximal end of the leading end body and is configured for detachable attachment to the trailing component. With this design, attachment or detachment of the leading and trailing components in the leadless pacemaker can be conveniently accomplished by coupling the first connection member to the delivery device and then, with the leading component being kept stationary by the delivery device, exerting a force on the trailing component by the delivery device. Moreover, during the attachment or detachment, immobilizing the leading component using the delivery device can avoid the leading component from pulling or retracting cardiac tissue to which the leading component is anchored, thus making the attachment or detachment safer. In this way, when a battery in the trailing component is exhausted, the leading component is allowed to remain in the heart, while the trailing component can be conveniently replaced with a new one using the delivery device without affecting the leading component at all.

The present invention will be described below with reference to the accompanying drawings.

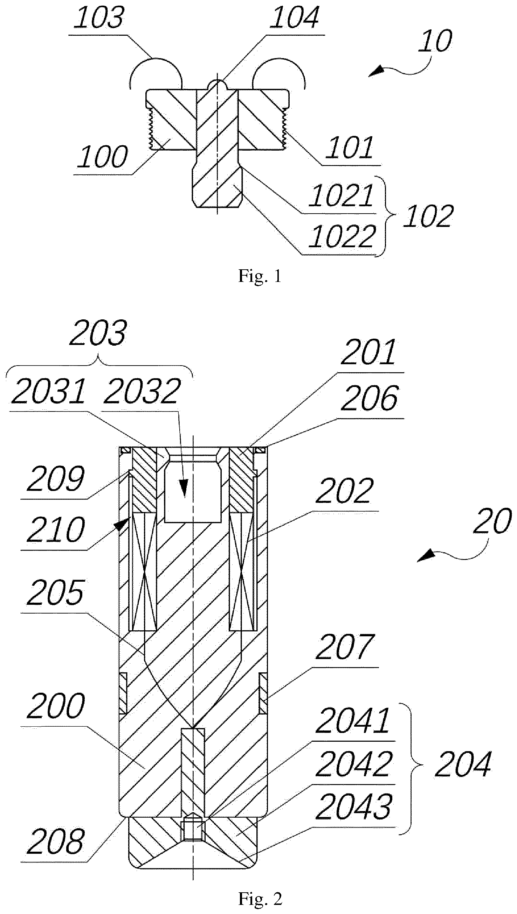

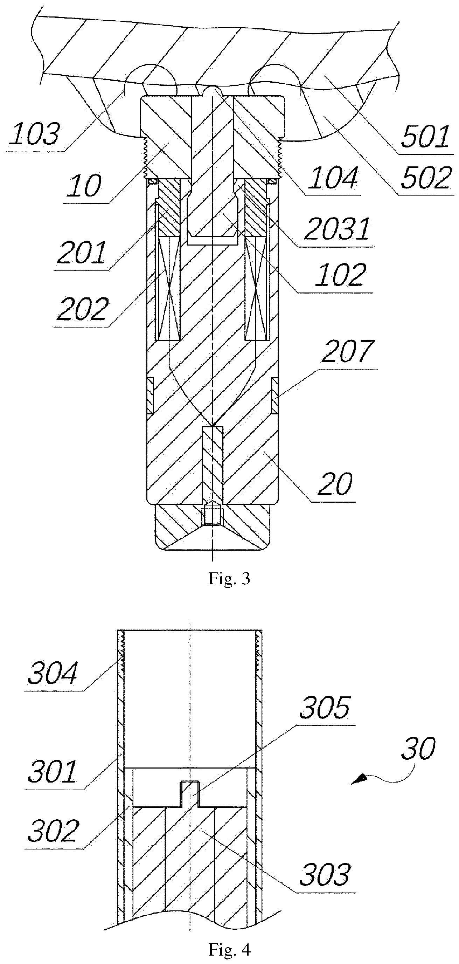

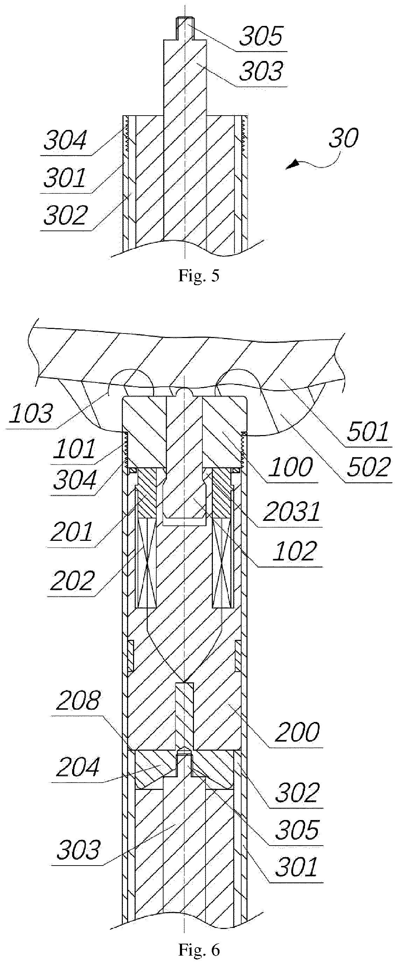

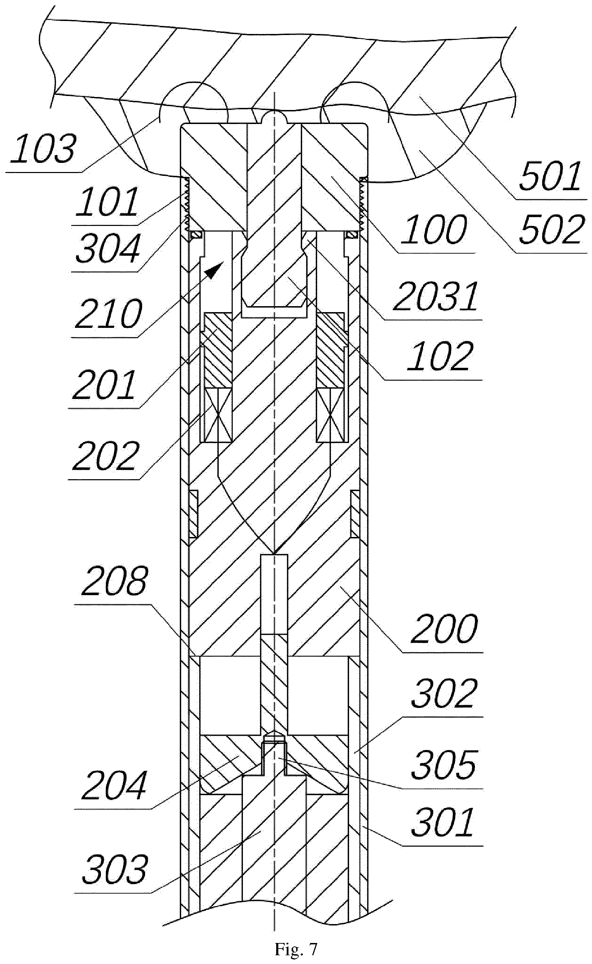

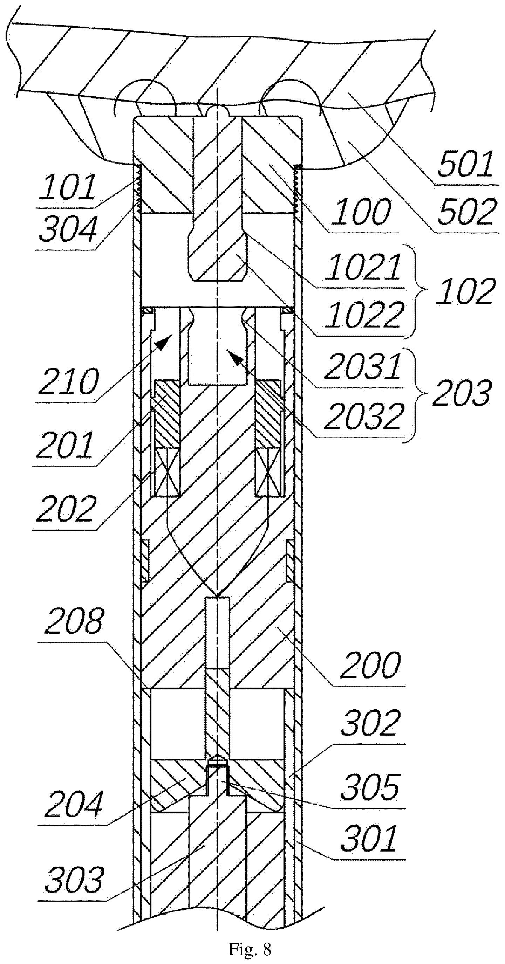

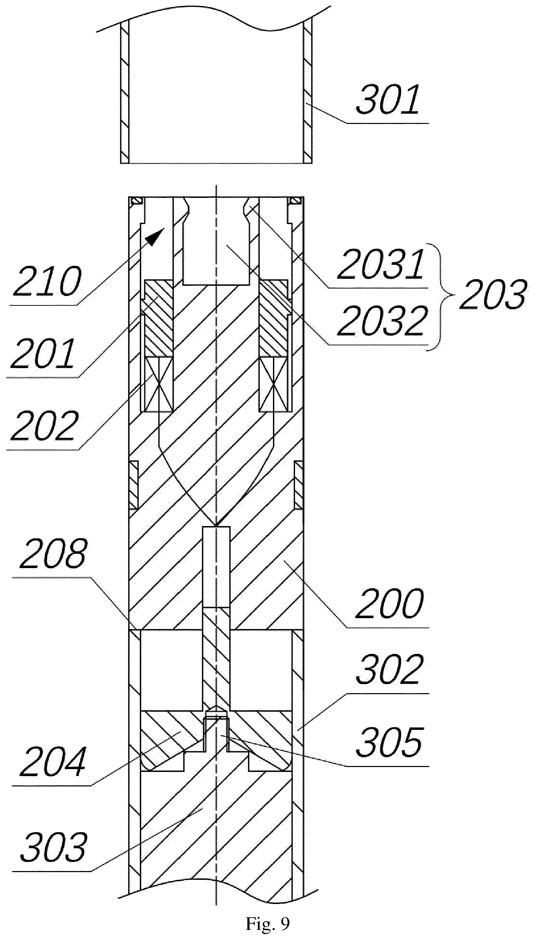

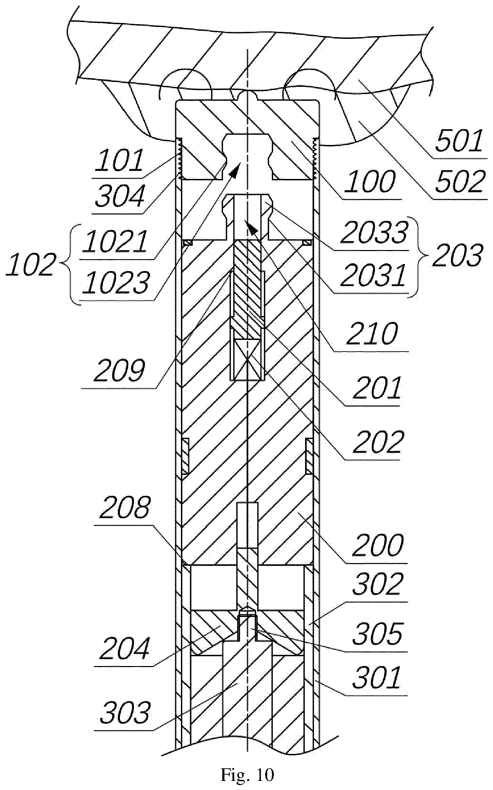

Reference is now made to Figs. 1 to 10. Fig. 1 is a schematic illustration of a leading component according to an embodiment of the present invention. Fig. 2 is a schematic illustration of a trailing component according to an embodiment of the present invention. Fig. 3 is a schematic illustration of a leadless pacemaker according to an embodiment of the present invention. Fig. 4 is a schematic illustration of a delivery device according to an embodiment of the present invention. Fig. 5 is another schematic illustration of the delivery device of Fig. 4 with a delivery rod thereof extending out. Fig. 6 schematically illustrates a first step in a trailing component replacement process performed by the delivery device according to an embodiment of the present invention. Fig. 7 schematically illustrates a second step in the trailing component replacement process of Fig. 6. Fig. 8 schematically illustrates a third step in the trailing component replacement process of Fig. 6. Fig. 9 schematically illustrates a fourth step in the trailing component replacement process of Fig. 6. Fig. 10 schematically illustrates the replacement of a trailing component in the leadless pacemaker according to another embodiment of the present invention.

As shown in Fig. 3, a leadless pacemaker according to an embodiment of the present invention includes a leading component 10 and a trailing component 20, the leading component 10 is detachably coupled to the trailing component 20. Referring to Fig. 1, in one exemplary embodiment, the leading component 10 includes a leading end body 100 provided with a fixation member 103 at a distal end of the leading end body 100. The fixation member 103 is configured to fix the leading end body 100 to a first object (e.g., cardiac tissue 501). One end of the fixation member 103 is fixed to the leading end body 100, and the other end is configured to pierce and anchor in the first object. Preferably, the fixation member 103 is shaped like a hook having a sharp free end that can easily pierce the first object such as the cardiac tissue 501. The leading end body 100 is also provided with an electrode 104 at the distal end, which is configured to abut against the cardiac tissue 501 and thus be capable of cardiac pacing or sensing when the fixation member 103 has pierced the cardiac tissue 501 and thus secured the leading end body 100 to the cardiac tissue 501. Generally, after the fixation member 103 has stayed in the cardiac tissue 501 for a certain period of time, proliferating tissue 502 will grow in the heart around the fixation member 103 and part of the leading end body 100, thus more reliably securing the leading component 10 within the heart. The trailing component 20 includes a battery. When the trailing component 20 is coupled to the leading component 10, the battery can power the whole leadless pacemaker and thus enable cardiac pacing or sensing of the electrode 104. When the battery is exhausted, a delivery device may be manipulated to easily decouple the trailing component 20 loaded with the battery from the leading component 10, thus allowing the trailing component 20 to be removed from the patient's body. A brand new trailing component 20 (including a new battery) may be then implanted using the delivery device and coupled to the leading component 10 so that they make up another complete leadless pacemaker that can instead provide the desired function. Thus, when the battery of the leadless pacemaker is exhausted, the connection relationship between the leading component 10 and the heart is allowed to remain intact, and the trailing component 20 is replaced by using the delivery device, so as to avoid disturbing the leading component 10 surrounded by the proliferating tissue 502. In this way, reuse of the leading component 10 is achieved, dispensing with the need to create a new incision in the heart.

Referring to Fig. 1, in conjunction with Fig. 4, a leading component 10 according to an embodiment of the present invention includes a first connection member 101 and a second connection member 102. The first connection member 101 is provided around the leading end body 100 and configured for detachable attachment to the delivery device 30 (in particular, to a first sheath 301 thereof). The attachment of the first connection member 101 to the delivery device 30 restricts at least axial movement of the first connection member 101 relative to the delivery device 30. The second connection member 102 is provided at a proximal end of the leading end body 100 and configured for detachable connection with a trailing component 20. The leading component 10 is so configured that, during its attachment or detachment to or from the trailing component 20, the attachment of the first connection member 101 to the delivery device 30 enables the leading component 10 to be held stationary axially by the delivery device 30. Holding the leading component 10 stationary axially by the delivery device 30 during attachment or detachment of the leading component 10 to or from the trailing component 20 avoids the leading component 10 pulling the cardiac tissue and thus making the attachment or detachment safer. Preferably, the first connection member 101 includes a first threaded section or a first circumferential snap fastener. The first threaded section may include, for example, a first external thread capable of engagement with the delivery device 30. The first circumferential snap fastener may include, for example, a circumferential snap groove or rib capable of circumferential engagement with the delivery device 30 and thereby restricting axial movement of the first connection member 101 relative to the delivery device 30, i.e., maintaining the first connection member 101 axially stationary relative to the delivery device 30.

Referring to Fig. 2, in conjunction with Figs. 3 and 4, a trailing component 20 according to an embodiment of the present invention includes a trailing end body 200, a third connection member 203 and a fourth connection member 204. The third connection member 203 is provided at a distal end of the trailing end body 200 and configured for detachable attachment to the leading component 10 (in particular, to the second connection member 102). The fourth connection member 204 is provided at a proximal end of the trailing end body 200 and configured for detachable attachment to the delivery device 30 (in particular, to a delivery rod 303 thereof). The fourth connection member 204 may be driven by the delivery device 30 to move along an axial direction of the trailing end body 200, thus causing attachment or detachment of the third connection member 203 to or from the leading component 10, with the leading component 10 being retained by the delivery device 30 to be axially stationary. In order to attach the trailing component 20 to the leading component 10, the fourth connection member 204 may be axially driven by the delivery device 30 to move distally, thus bringing the third connection member 203 into engagement with the leading component 10. In order to detach the trailing component 20 from the leading component 10, the fourth connection member 204 may be axially driven by the delivery device 30 to move proximally, thus disengaging the third connection member 203 from the leading component 10.

In a preferably embodiment, the second connection member 102 includes a first projection 1022 projecting from a proximal end face of the leading end body 100, which is configured to mate with a corresponding connection structure of the trailing component 20 and locate the trailing component 20. The third connection member 203 includes a second cavity 2032 provided at the distal end of the trailing end body 200. The second cavity 2032 can be mated with the first projection 1022 in such a manner that the first projection 1022 can be inserted and fitted in the second cavity 2032. That is, the second cavity 2032 is just said corresponding connection structure for the first projection 1022. Preferably, the second connection member 102 further includes a first locking element 1021 provided on a side wall of the first projection 1022, the first locking element 1021 is configured to lock the trailing component 20. The third connection member 203 further includes a second locking element 2031 provided on a side wall of the second cavity 2032, the second locking element 2031 is configured to lock the leading component 10. More specifically, the second cavity 2032 is provided primarily to receive the first projection 1022 of the second connection member 102. It is adapted in shape to the first projection. For example, both of them may be substantially cylindrical. The second cavity 2032 may be a blind bore having an opening at its distal end, from which the first projection 1022 can be inserted. The second locking element 2031 is preferably annular. More preferably, a plurality of such second locking elements 2031 are provided, each of which extends circumferentially with respect to the second cavity 2032. One of the first locking element 1021 and the second locking element 2031 has a snap groove, and the other has a snap rib that can fit in the snap groove. For example, in an optional embodiment, the second locking element 2031 may define an inwardly-protruding snap rib around its distal end, and the first locking element 1021 may define a corresponding snap groove around its distal end, which can fit with the snap rib. As shown in Fig. 1, the snap groove may be defined by both a distal first bevel of the first projection 1022 and the leading end body 100. In alternative embodiments, it is also possible to provide a snap groove in the second locking element 2031 and a corresponding snap rib in the first locking element 1021. Those skilled in the art can select a suitable arrangement depending on the circumstance. Preferably, the proximal end of the first projection 1022 is provided with a second bevel, which is inclined inwardly proximally in order to facilitate alignment and insertion of the first projection 1022 with and into the second cavity 2032. Axially driving the first locking element 1021 and the second locking element 2031 toward each other (e.g., with the leading component 10 being held stationary at the cardiac tissue, driving the trailing component 20 to move axially distally) will result in engagement of them and abutment of the leading end body 100 against the trailing component 20. On the contrary, when the first locking element 1021 and the second locking element 2031 are axially driven opposite to each other (e.g., with the leading component 10 being held stationary at the cardiac tissue, the trailing component 20 is driven to move axially proximally), the second locking element 2031 will elastically deform under the action of a pressure exerted by the first bevel of the first locking element 1021 and widen the aforementioned opening, thus allowing disengagement of the first locking element 1021 from the second locking element 2031. Optionally, a sealing ring 206 may be provided at the distal end of the trailing end body 200 so as to be close to an outer periphery of the end face. In this case, when the first locking element 1021 and the second locking element 2031 are engaged and locked with each other, the trailing end body 200 is sealed against the leading end body 100 by the sealing ring 206, preventing egress of liquid to the leadless pacemaker. Optionally, both the first locking element 1021 and the second locking element 2031 may be electrical conductors. Alternatively, the first projection 1022 and a wall defining the second cavity 2032 may be electrical conductors. In these cases, the leading component 10 may be electrically connected the trailing component 20 when the first locking element 1021 is brought into contact with the second locking element 2031, or when the first projection 1022 is brought into contact with the wall defining the second cavity 2032. In an alternative embodiment, electronic elements of the leadless pacemaker (including, but not limited to those necessary for operation of the leadless pacemaker, such as an impulse generator, electronic communication elements, an operational processor, etc.) and the battery are all disposed within the trailing component 20, and a sensing electrode 207 with sensing capabilities coupled to one or more of the electronic elements is provided on an outer circumference of the trailing component 20. The electrode 104 of the leading component 10 may be a pacing electrode. The electrical connection between the leading component 10 and the trailing component 20 enables transmission of cardiac pacing signals from one of the electronic elements in the trailing component 20 to the electrode 104 in the leading component 10. It is to be noted that all or some of the electronic elements in the leadless pacemaker may be disposed in one of the leading component 10 and the trailing component 20. The electrical connection between the leading component 10 and the trailing component 20 enables transmission of pacing signals to the leading component 10 so that they can effect cardiac pacing via the electrode 104. Alternatively, the electrode 104 may further have sensing capabilities, and signals sensed thereby may be transmitted to one or more of the electronic elements. Those skilled in the art may select a suitable arrangement as required.

The trailing component 20 further includes a limiting block 201 movably disposed within the trailing end body 200. The limiting block 201 is configured to the second locking element 2031 so that the leading component 10 is locked by the second locking element 2031. Preferably, the limiting block 201 is coupled to the fourth connection member 204. The fourth connection member 204 is configured to drive the limiting block 201 to move axially within the trailing end body 200 so as to cause the limiting block 201 to limit the second locking element 2031 or not. When urged by the delivery device 30 axially with respect to the trailing end body 200, the fourth connection member 204 can drive the limiting block 201 to move. Specifically, when the delivery device 30 drives the fourth connection member 204 axially away from the trailing end body 200, the limiting block 201 will not limit the second locking element 2031 any longer, thus allowing the trailing component 20 to be detached from the leading component 10. Preferably, the trailing end body 200 further includes an axial-extending slide channel 210, in which the limiting block 201 is disposed so as to be movable axially with respect to the trailing end body 200. The slide channel 210 circumferentially surrounds the second locking element 2031, and is flush with the second locking element 2031 distally. The limiting block 201 is adapted in shape to the slide channel 210. When the limiting block 201 is distally aligned with the slide channel 210, the second locking element 2031 is surrounded by the limiting block 201 and thus deprived of its freedom of radial movement. In this configuration, the leading component 10 is locked by the second locking element 2031. More specifically, when the limiting block 201 is aligned distally with the slide channel 210, radial deformation of the second locking element 2031 is limited by the limiting block 201, and the first projection 1022 is therefore locked within the second cavity 2032, thus resulting in reliable attachment of the leading component 10 to the trailing component 20. When the limiting block 201 is proximally moved away from the distal end of the slide channel 210, the second locking element 2031 will be circumferentially exposed in the slide channel 210 and its distal end portion thus becomes free to deform radially. In this configuration, the second locking element 2031 is no longer limited, and when opposite axial forces are applied to the first and second locking elements 1021 and 2031, the second locking element 2031 will deform outwardly radially. In this way, the first locking element 1021 and second locking element 2031 can be disengaged from each other, allowing detachment of the leading component 10 from the trailing component 20. Further, the slide channel 210 may define a first limiting surface 209, which is configured to abut against the limiting block 201 and thus prevent it axially move beyond the distal end of the trailing end body 200. The first limiting surface 209 may be, for example, a step surface facing proximally. Correspondingly, the limiting block 201 may define another step surface facing distally. These opposite facing step surfaces may be complementary in shape. When the limiting block 201 is moving axially distally, upon the two step surfaces coming into abutment against each other, the first limiting surface 209 will disallow further axial distal movement of the limiting block 201. In this way, the limiting block 201 will not axially protrude out of the trailing end body 200 distally, resulting in better sealing between the leading component 10 and the trailing component 20 when they are engaged together.

Referring to Fig. 10, in another embodiment, the second connection member 102 defines a first cavity 1023 recessed from the proximal end face of the leading end body 100, which is configured to mate with a corresponding connection structure of the trailing component 20 and thus locate the trailing component 20. The third connection member 203 includes a second projection 2033 projecting from the distal end face of the trailing end body 200, the first cavity 1023 is mateable with the second projection 2033 in such a manner that the second projection 2033 can be inserted and fitted in the first cavity 1023. That is, the second projection 2033 is just said corresponding connection structure for the first cavity 1023. Optionally, the second projection 2033 and a wall defining the first cavity 1023 are both electrical conductors. In this case, the leading component 10 will be electrically connected to the trailing component 20 upon the wall defining the first cavity 1023 coming into contact with the second projection 2033. Moreover, a first locking element 1021 is provided on a side wall of the first cavity 1023, and a second locking element 2031 on a side wall of the second projection 2033. A limiting block 201 is movably disposed in the second projection 2033 in order to limit the second locking element 2031. Further, an axially-extending slide channel 210 is defined in the second projection 2033, and the limiting block 201 is disposed in the slide channel 210 so as to be movable therein. Furthermore, the slide channel 210 defines a first limiting surface 209, which is configured to abut against the limiting block 201 and thus prevent the limiting block 201 from axially protruding out of the second projection 2033 distally.

Preferably, the fourth connection member 204 is able to move axially with respect to the trailing end body 200 so as to come into connection with the trailing end body 200. The trailing end body 200 includes a second limiting surface 208, which is configured to abut against the delivery device 30 (in particular, a second sheath 302 thereof), thus limiting axial proximal movement of the trailing end body 200. The limiting block 201 is configured to move proximally when the fourth connection member 204 is axially proximally driven by the delivery device 30 during abutment of the second limiting surface 208 against the delivery device 30, thus no longer limiting the second locking element 2031. Preferably, the fourth connection member 204 includes a member body 2042, the member body 2042 is axially movable with respect to the trailing end body 200. For example, the trailing end body 200 may define a receptacle at its proximal end, and the member body 2042 may define at its distal end a guide stud that matches the receptacle. Inserting the guide stud within the receptacle deprives freedom of radial movement of the member body 2042. The Fourth connection member 204 may further include a second threaded section 2041 or a second circumferential snap fastener. The second threaded section 2041 may include a second internal thread and be open proximally in order to allow for connection of a corresponding connection structure (in particular, a third threaded section 304) of the delivery device 30. Alternatively, the second circumferential snap fastener may include a circumferential snap groove or rib capable of circumferential engagement with the delivery device 30. Such engagement can restrict axial movement of the delivery device 30 and the fourth connection member 204, i.e., immobilizing the fourth connection member 204 axially relative to the delivery device 30. More preferably, the fourth connection member 204 further includes a guide slope 2043, which is provided at the proximal end of the member body 2042 and flared proximally in order to facilitate connection of an end portion of the delivery device 30 to the second threaded section 2041. Optionally, the limiting block 201 may be tied to the fourth connection member 204 with a flexible thread 205. Specifically, the tying by the flexible thread 205 may be accomplished at both a proximal end of the limiting block 201 and a distal end of the member body 2042. When the trailing end body 200 is restricted from moving axially proximally, axially proximally moving the fourth connection member 204 will tighten the flexible thread 205 and thus pull the limiting block 201 to move proximally within the slide channel 210 away from the position where it limits the second locking element 2031.

The trailing component 20 further includes a first force-applying element 202 configured to exert a distal first force on the limiting block 201 and thereby cause the limiting block 201 to limit the second locking element 2031. In order to displace the limiting block 201 from the position where it limits the second locking element 2031, the fourth connection member 204 must overcome the first force. Specifically, when the delivery device 30 proximally exerts on the fourth connection member 204 an axial force greater than the first force, the limiting block 201 will be displaced and no longer limit the second locking element 2031. Any axial proximal force applied by the delivery device 30 to the fourth connection member 204 that is less than the first force cannot move the limiting block 201 away from the second locking element 2031. Under the action of the distal first force exerted by the first force-applying element 202 on the limiting block 201, the limiting block 201 is position by default at a distal end of the slide channel 210. During normal use of the leadless pacemaker in a patient, both the first connection member 101 and the fourth connection member 204 are unsupported and not connected to the delivery device 30. In this configuration, the limiting block 201 always limits the second locking element 2031 under the action of the first force from the first force-applying element 202 and thus ensures reliable attachment between the leading component 10 and the trailing component 20 during normal use of the leadless pacemaker. In order to replace the trailing component 20 in the event of depletion of the battery therein, the delivery device 30 is coupled to the fourth connection member 204 and proximally exerts thereon an axial force exceeding the first force. As a result, the fourth connection member 204 overcomes the first force from the first force-applying element 202 and causes the limiting block 201 to move proximally through dragging it with the flexible thread 205. After the limiting block 201 moves away, the second locking element 2031 is no longer limited. However, when the axial proximal force exerted on the fourth connection member 204 by the delivery device 30 decreases below the first force (or the fourth connection member 204 is not subjected to the axial force by the delivery device 30), the limiting block 201 will be driven by the first force-applying element 202 to move distally and will again limit the second locking element 2031. In this case, optionally, the fourth connection member 204 may be dragged by the flexible thread 205 to also move distally until the member body 2042 abuts against the trailing end body 200. Optionally, the first force-applying element 202 may be an elastic element such as a spring, shrapnel or the like.

Referring to Figs. 4 and 5, in an embodiment of the present invention, there is provided a delivery device 30 for delivering the above-described leading component 10, trailing component 20 or leadless pacemaker. The delivery device 30 includes a first sheath 301 and a delivery rod 303. The first sheath 301 is configured to be coupled to the first connection member 101 of the leading component 10 in order to maintain the leading component 10 axially stationary. The delivery rod 303 is disposed within the first sheath 301 and is configured to be coupled to the fourth connection member 204 of the trailing component 20 in order to attach the trailing component to the leading component or detach them from each other. Specifically, in order to attach the leading component 10 to the trailing component 20, the delivery device 30 is configured with the first sheath 301 being coupled to the first connection member 101 and held axially stationary so that the leading component 10 will not move in this direction. The delivery rod 303 is then moved axially distally to attach the trailing component 20 to the leading component 10. In order to detach the trailing component 20 from the leading component 10, the delivery device 30 is configured with first sheath 301 being coupled to the first connection member 101 and held axially stationary so that the leading component 10 will not move in this direction. The delivery rod 303 is then moved axially proximally to detach the trailing component 20 from the leading component 10. Preferably, in order to facilitate the delivery and assembly of the leadless pacemaker, both the first sheath 301 and the delivery rod 303 in the delivery device 30 may have freedom of axial translation and circumferential rotation, which enables them to axially translate and circumferentially rotate relative to each other. Coupling the first sheath 301 in the delivery device 30 to the first connection member 101 enables the first sheath 301 to axially immobilize the leading component 10 during the attachment or detachment and thus prevent it from pulling or retracting the cardiac tissue. This makes the attachment or detachment safer. In this way, when the battery in the trailing component 20 is exhausted, the leading component 10 is allowed to remain in the heart, while the trailing component 20 can be conveniently replaced using the delivery device 30 without affecting the leading component 10 at all.

Preferably, the delivery device 30 further includes a second sheath 302 disposed between the first sheath 301 and the delivery rod 303. The second sheath 302 is configured to abut against the trailing end body 200 (e.g., against the second limiting surface 208 thereof) to limit the axial proximal movement of the trailing end body 200. Preferably, the second sheath 302 is movable at least axially so as to be able to come into abutment against the trailing end body 200 or move from contact therewith.

Optionally, a distal end of the first sheath 301 may have a third threaded section 304, the third the threaded section 304 includes a third internal thread that is engageable with the first external thread of the first threaded section of the first connection member 101. In this way, rotating the first sheath 301 will result in attachment or detachment of the first connection member 101. Engaging the first sheath 301 with the first connection member 101 will restrict axial displacement of the first sheath 301 relative to the leading component 10. In some other embodiments, the distal end of the first sheath 301 may be provided with a third circumferential snap fastener that is engageable with the first circumferential snap fastener on the leading component 10. For example, the first circumferential snap fastener may be implemented as a circumferential snap groove, and the third circumferential snap fastener as a snap rib which can circumferentially snap into the snap groove, thus restricting axial displacement of the first sheath 301 relative to the leading component 10. Of course, it is also possible that the first circumferential snap fastener is implemented as a circumferential snap rib and the third circumferential snap fastener as a circumferential snap groove. These circumferential snap fasteners also allow fast and easy attachment or detachment of the first sheath 301 and the first connection member 101, and their mutual engagement will restrict axial movement of the two.

Optionally, the distal end of the delivery rod 303 may have a fourth threaded section 305, the fourth threaded section 305 includes a fourth external thread that is engageable with the second internal thread of the second threaded section 2041 of the fourth connection member 204. In this way, rotating the delivery rod 303 will result in attachment or detachment of the fourth connection member 204. Engaging the delivery rod 303 with the fourth connection member 204 will restrict axial displacement of the delivery rod 303 relative to the fourth connection member 204. In some other embodiments, the distal end of the delivery rod 303 may be provided with a fourth circumferential snap fastener that is engageable with the second circumferential snap fastener on the fourth connection member 204. One can refer to the above description of the third and first circumferential snap fasteners for more details in the structure and working mechanism of the fourth and second circumferential snap fasteners.

On the basis of the above-described configurations and arrangements, the delivery device 30 is able to deliver the leading component 10 and the trailing component 20 individually, or the entire leadless pacemaker. For a patient who is to receive cardiac pacemaker implantation for the first time, it is either possible to implant the entire leadless pacemaker in one pass, or to implant the leading component 10 in a first step and then implant the trailing component 20 and attach it to the leading component 10 in a second step. Subsequently, when a need is identified for replacing the trailing component 20, a new trailing component 20 may be implanted separately.

Steps in a process for replacing the trailing component 20 using the delivery device 30 according to an embodiment of the present invention will be explained in detail below with reference to Figs. 5 to 9.

In step S1, as shown in Fig. 5, the delivery device 30 is delivered into the heart, and the delivery rod 303 is caused to extend distally so that the fourth threaded section 305 is exposed.

In step S2, the delivery rod 303 is rotated to engage the fourth threaded section 305 with the second threaded section 2041 of the fourth connection member 204 in the trailing component 20.

In step S3, with the delivery rod 303 being held stationary axially, the delivery rod 303 is manipulated to distally push the first sheath 301 and the second sheath 302 until the distal end of the first sheath 301 reaches the leading end body 100 of the leading component 10 and the distal end of the second sheath 302 reaches the trailing end body 200 of the trailing component 20.

In step S4, the first sheath 301 is rotated to engage the third threaded section 304 of the first sheath 301 with the first threaded section of the first connection member 101, as shown in Fig. 6.

In step S5, with the first sheath 301 and the second sheath 302 being held stationary axially, the delivery rod 303 is proximally retracted with a force exceeding the first force of the first force-applying element 202 so that both the fourth connection member 204 and the limiting block 201 moves proximally and the second locking element 2031 is no longer limited, as shown in Fig. 7.

In step S6, the second sheath 302 is not held stationary any longer and is pulled proximally together with the delivery rod 303 so that the first bevel of the first locking element 1021 forces the second locking element 2031 to deform outwardly. The first projection 1022 is then removed from the second cavity 2032, detaching the trailing component 20 from the leading component 10.

In step S7, the second sheath 302 and the delivery rod 303 are further proximally retracted until the trailing component 20 is withdrawn from the patient's body. The delivery rod 303 is again caused to extend distally so that the fourth threaded section 305 is exposed. A new trailing component 20 is then engaged with the delivery rod 303, and the second sheath 302 is moved into abutment against the new trailing component 20. The delivery rod 303 is then retracted proximally with a force exceeding the first force of the first force-applying element 202. As a result, both the fourth connection member 204 and the limiting block 201 move proximally so that the second locking element 2031 is not limited any more, as shown in Fig. 9.

In step S8, with the second sheath 302 and the delivery rod 303 being retained axially stationary relative to each other, the new trailing component 20, together with the second sheath 302 and the delivery rod 303, is inserted into the distal end of the first sheath 301. The second sheath 302 and the delivery rod 303 are then pushed distally until the configuration shown in Fig. 8 is reached. The second sheath 302 and the delivery rod 303 and then further pushed until the first projection 1022 is inserted into the second cavity 2032 and a snap fit is created between the first locking element 1021 and the second locking element 2031, as shown in Fig. 7.

In step S9, the second sheath 302 and the delivery rod 303 are no longer retained axially stationary relative to each other, and the delivery rod 303 is distally pushed to cause the limiting block 201 move distally until the limiting block 201 is limited by the first limiting surface 209, as shown in Fig. 6.

In step S10, the first sheath 301 is rotated in the opposite direction and is thus disengaged from the first connection member 101. The delivery rod 303 is then rotated in the opposite direction and is thereby disengaged from the fourth connection member 204. The first sheath 301, the second sheath 302 and the delivery rod 303 are all withdrawn, ending the replacement of the trailing component 20.

In summary, the present invention provides a leadless pacemaker, a leading component, a trailing component and a delivery device. The leading component includes a leading end body, a first connection member and a second connection member. The trailing component includes a trailing end body, a third connection member and a fourth connection member. Attachment or detachment of the leading and trailing components of the leadless pacemaker can be easily accomplished by coupling the first connection member to the delivery device and then, with the leading component being axially immobilized by the delivery device, coupling or decoupling the second connection member to or from the third connection member under the action of an axial force exerted on the fourth connection member by the delivery device. During the attachment or detachment, through axially immobilizing the leading component using the delivery device, pulling or retracting of cardiac tissue to which the leading component is anchored is avoided, making the attachment or detachment safer. In this way, when a battery in the trailing component is exhausted, the leading component is allowed to remain in the heart, while the trailing component can be conveniently replaced with a new one using the delivery device without affecting the leading component at all.

It is to be noted that the foregoing embodiments are merely exemplary of the present invention and do not limit the scope thereof in any way. Although various variations and modifications can be made by those of ordinary skill in the art without departing from the concept of the present invention, any and all such variations and modifications are intended to be embraced within the scope of the invention as defined by the appended claims.

## Claims

1. A leading component of a leadless pacemaker, configured for detachably connectable to a trailing component of the leadless pacemaker, the leading component comprising:
a leading end body configured for anchoring to a first object;
a first connection member disposed on an outer periphery of the leading end body, the first connection member configured for detachably connectable to a delivery device; and
a second connection member disposed at a proximal end of the leading end body, the second connection member configured for detachably connectable to the trailing component.

2. The leading component according to claim 1, wherein when the first connection member is connected to the delivery device, at least axial movement of the first connection member relative to the delivery device is limited.

3. The leading component according to claim 1 or 2, wherein the first connection member comprises a first threaded section or a first circumferential snap fastener.

4. The leading component according to claim 1, wherein the second connection member comprises a first projection projecting from a proximal end face of the leading end body or a first cavity provided at the proximal end of the leading end body, the first projection or the first cavity configured to mate with a corresponding connection structure of the trailing component.

5. The leading component according to claim 4, wherein the second connection member further comprises a first locking element provided on a side wall of the first projection or on a side wall of the first cavity.

6. The leading component according to claim 1, further comprising a fixation member, which is fixed to the leading end body at one end and configured to pierce and anchor in the first object at the other end.

7. A trailing component of a leadless pacemaker, configured for detachably connectable to a leading component of the leadless pacemaker, the trailing component comprising:
a trailing end body;
a third connection member disposed at a distal end of the trailing end body, the third connection member configured for detachably connectable to the leading component; and
a fourth connection member disposed at a proximal end of the trailing end body, the fourth connection member configured for detachably connectable to a delivery device,
the fourth connection member configured to be able to move in an axial direction of the trailing end body relative to the third connection member when driven by the delivery device.

8. The trailing component according to claim 7, wherein the third connection member comprises a second projection projecting from a distal end of the trailing end body or a second cavity provided at the distal end of the trailing end body, the second projection or the second cavity configured to mate with a corresponding connection structure of the leading component.

9. The trailing component according to claim 8, wherein the third connection member further comprises a second locking element provided on a side wall of the second projection or on a side wall of the second cavity, the second locking element configured to lock the leading component.

10. The trailing component according to claim 9, wherein the locking element is annular.

11. The trailing component according to claim 9, further comprising a limiting block, wherein in the case of the third connection member comprising the second cavity, the limiting block is movably disposed in the trailing end body; or in the case of the third connection member comprising the second projection, the limiting block is movably disposed in the second projection, and wherein the limiting block is configured to limit the second locking element.

12. The trailing component according to claim 11, wherein the limiting block is connected to the fourth connection member so that the fourth connection member is able to drive the limiting block to move in the axial direction of the trailing end body to control the limiting block to apply or release the limiting of the locking element.

13. The trailing component according to claim 12, wherein the fourth connection member is connected with the trailing end body, and wherein the fourth connection member is movable in the axial direction of the trailing end body and configured to drive the limiting block to move under the action of an axial force exerted by the delivery device.

14. The trailing component according to claim 13, wherein when the axial force is exerted on the fourth connection member by the delivery device away from the trailing end body, the limiting block releases the limiting of the locking element.

15. The trailing component according to claim 11, wherein the limiting block is connected to the fourth connection member by a flexible thread.

16. The trailing component according to claim 11, further comprising a first force-applying element configured to exert a first force towards the distal end to the limiting block, wherein the limiting block limits the locking element under the action of the first force, and wherein the limiting block overcomes the first force to release the limiting of the locking element driven by the fourth connection member.

17. The trailing component according to claim 16, wherein the first force-applying element is an elastic element.

18. The trailing component according to claim 11, wherein the trailing end body or the second projection is further provided with a slide channel arranged along the axial direction, and wherein the limiting block is slidably disposed in the slide channel.

19. The trailing component according to claim 18, wherein the slide channel is provided with a first limiting surface, and wherein in the case of the third connection member comprising the second cavity, the trailing end body is provided with the slide channel arranged along the axial direction, and the first limiting surface is configured to abut against the limiting block and thus prevent the limiting block from axially moving beyond the distal end of the trailing end body;
or in the case of the third connection member comprising the second projection, the second projection is provided with the slide channel arranged along the axial direction, and the first limiting surface is configured to abut against the limiting block and thus prevent the limiting block from axially moving beyond a distal end of the second projection.

20. The trailing component according to claim 7, wherein the fourth connection member comprises a second threaded section or a second circumferential snap fastener.

21. A leadless pacemaker, comprising a leading component and a trailing component,
the leading component comprising a leading end body, a first connection member and a second connection member, the leading end body configured for anchoring to a first object, the first connection member disposed on an outer periphery of the leading end body and configured for detachably connectable to a delivery device, the second connection member disposed at a proximal end of the leading end body,
the trailing component comprising a trailing end body, a third connection member and a fourth connection member, the third connection member disposed at a distal end of the trailing end body, the fourth connection member disposed at a proximal end of the trailing end body and configured for detachably connectable to the delivery device, the fourth connection member configured to be able to move in an axial direction of the trailing end body relative to the third connection member when driven by the delivery device,
wherein the leading component is detachably connectable to the trailing component through the second and third connection members.

22. The leadless pacemaker according to claim 21, wherein the second connection member of the leading component comprises a first projection, and the third connection member of the trailing component comprises a second cavity adapted to receive the first projection, or wherein the second connection member comprises a first cavity and the third connection member of the trailing component comprises a second projection adapted to be received in the first cavity.

23. The leadless pacemaker according to claim 22, wherein the second connection member further comprises a first locking element provided on a side wall of the first projection or on a side wall of the first cavity, wherein the third connection member further comprises a second locking element provided on a side wall of the second projection or on a side wall of the second cavity, and wherein one of the first and second locking elements has a snap groove, and the other of the first and second locking elements has a snap rib configured to mate with the snap groove.

24. The leadless pacemaker according to claim 23, wherein the first and second locking elements are electrical conductors, or wherein the first projection and the second cavity are electrical conductors, or wherein the second projection and the first cavity are electrical conductors.

25. A delivery device for delivering the leading component according to any one of claims 1 to 6, the trailing component according to any one of claims 7 to 20, or the leadless pacemaker according to any one of claims 21 to 24, the delivery device comprising:
a first sheath configured for connecting with the first connection member of the leading component, the first sheath configured to axially immobilize the leading component; and
a delivery rod disposed inside the first sheath, the delivery rod configured for connecting with the fourth connection member of the trailing component, the delivery rod configured to connect the trailing component with the leading component or separate the trailing component from the leading component.

26. The delivery device according to claim 25, further comprising a second sheath disposed between the first sheath and the delivery rod, the second sheath configured to abut against the trailing end body to limit the axial movement of the trailing end body toward the proximal end thereof.
